Europäisches Patentamt

European Patent Office

Office européen des brevets

(11) Publication number: **0 047 473**
**B1**

(12) **EUROPEAN PATENT SPECIFICATION**

(45) Date of publication of patent specification: **02.03.88**

(51) Int. Cl.⁴: **C 07 D 301/02**

(21) Application number: **81106830.3**

(22) Date of filing: **01.09.81**

(54) Process for the preparation of epoxides from alkylene carbonates.

(30) Priority: **04.09.80 US 184067**

(43) Date of publication of application:
**17.03.82 Bulletin 82/11**

(45) Publication of the grant of the patent:
**02.03.88 Bulletin 88/09**

(84) Designated Contracting States:
**BE DE FR GB IT NL**

(56) References cited:
**DE-A-2 855 232**
**DE-C- 845 937**
**US-A-2 851 469**
**US-A-4 069 234**

**J. Org. Chem., USSR, no. 5(2), 1969, p. 222;
SHAPIRO et al.**

(73) Proprietor: **UNION CARBIDE CORPORATION**
**39 Old Ridgebury Road**
**Danbury Connecticut 06817 (US)**

(72) Inventor: **McMullen, Charles Henry**
**44 Sunrise Avenue**
**Katonah (10536) New York (US)**

(74) Representative: **Wuesthoff, Franz, Dr.-Ing. et al
Patentanwälte Wuesthoff -v. Pechmann-
Behrens-Goetz Schweigerstrasse 2
D-8000 München 90 (DE)**

Courier Press, Leamington Spa, England.

## Description

This invention relates to a process for converting alkylene carbonates to their corresponding epoxides. More particularly, this invention is concerned with a process for generating an alkylene oxide such as ethylene oxide from the corresponding alkylene carbonate with a minimum formation of undesired aldehyde and/or ketone byproducts.

Processes for the efficient conversion of alkylene carbonates, particularly ethylene and propylene carbonates, to the corresponding epoxides (namely, ethylene oxide and propylene oxide) have heretofore been the focus of relatively limited study in the prior art. Processes to synthesize the alkylene carbonate starting material from an alkylene oxide and carbon dioxide are more common in the art and are amply described in the patent literature. U.S. Patents Nos. 2,773,070; 2,873,282; 2,907,771; and 2,994,704 are descriptive of such carbonate-forming process. Belgian patent No. 872,960 is directed to a particularly efficient process for alkylene carbonate formation which is characterized by alkylene carbonate efficiencies above 99% and an epoxide conversion of greater than 99.5%. In view of the relatively advanced state of the art of alkylene carbonate formation as reflected by the patents disclosed above, a process which efficiently converts an alkylene carbonate, such as ethylene carbonate, to alkylene oxide could be advantageously combined with an efficient alkylene carbonate forming process so as to allow ethylene carbonate to be used as an economical, safe and non-explosive medium of transportation for ethylene oxide. The ethylene oxide could thus be efficiently generated from ethylene carbonate upon demand and thereby avoid the inherent hazards of shipping and storing the flammable oxide prior to use. Moreover, in instances where the reactant alkylene carbonate is produced from non-epoxide starting materials, such carbonate would represent a particularly desirable source for generating alkylene oxide.

From DE—A—845 937, the production of alkylene oxides by reacting alkylene carbonates at a temperature of 100 to 200°C under normal or reduced pressure is known, the reaction being carried out substantially in an inert atmosphere and leading to an alkylene oxide which is to serve as pesticide. No information as to the portion to by-products of the reaction product can be taken from this prior art. It is also not apparent why operation is to be carried out at reduced pressure.

From US—A—4 069 234 it is known to produce vicinal epoxides by heating the corresponding alkylene carbonate in the presence of a catalyst at any suitable pressure ranging from subatmospheric to—preferably—atmospheric. The catalyst is a phosphonium halide, sulfonium halide, sulfoxonium halide or a metal salt—as the halide, sulfate and carboxylate of iron, tin, manganese and zinc.

A major source of inefficiency in the conversion of an alkylene carbonate to an alkylene oxide and carbon dioxide is the formation of aldehydes and/or ketones as by-products of the reaction. For processes where the alkylene carbonate reactant is, for example, ethylene carbonate, acetaldehyde is generally formed as an undesired by-product. Similarly, the conversion of propylene carbonate usually results in the undesired formation of propionaldehyde and/or acetone; the particular aldehyde and/or ketone thus produced being dependent upon the choice of alkylene carbonate reactant. The presence of such aldehyde and ketone by-products is deemed unacceptable in commercial grade alkylene oxides because they adversely affect the products ultimately formed by the catalyzed reactions of such alkylene oxides. Moreover, the separation of such aldehydes and ketones from alkylene oxide is troublesome and costly. For example, the separation of acetaldehyde from ethylene oxide is a relatively difficult operation inasmuch as both materials are liquids having relatively similar volatilities. As a consequence, separation is usually effected in commercial operation by a relatively elaborate fractional distillation.

## Summary of the invention

The invention describes a homogeneous catalytic process for converting an alkylene carbonate to the corresponding epoxide wherein a liquid phase containing the alkylene carbonate and a soluble catalyst is reacted by heating to a temperature of from 100 to 250°C at subatmospheric pressure to form a product mixture comprising predominantly alkylene oxide and carbon dioxide. The process is characterized by the improvement which comprises heating the liquid phase at a pressure between 39.9 and 266 mbar (30 to 200 mmHg) in the presence of from 0.001 to 0.1 mole/l of an alkali halide as the catalyst.

The present invention is predicated on the discovery that the formation of undesired aldehyde and/or ketone by-products of the reaction can be substantially reduced by carrying out the reaction under the conditions defined above. Although the mechanism by which such undesired by-products are minimized is not fully understood, it is believed that the effect of operating under said conditions is to minimize the residence time of alkylene oxide in the liquid phase. The availability of such alkylene oxide to interact with alkylene carbonate and the homogeneous catalyst is therefore correspondingly reduced thereby preventing the undesired side reactions which result in the formation of aldehyde and/or ketone by-products.

## Detailed description of the invention

The preferred alkylene carbonates contemplated by the invention are ethylene carbonate and propylene carbonate, such carbonates being reacted by the process of the invention to produce ethylene oxide and propylene oxide, respectively. Other alkylene carbonates may, however, be also advantageously used in accordance with the invention to produce the corresponding epoxide.

**0 047 473**

The invention contemplates the utilization of known alkali metal halide catalysts, such as described in Shapiro et al., J. Org. Chem. U.S.S.R., 5(2), p. 222 (1969), for the conversion of alkylene carbonate to alkylene oxide. Thus, the catalyst may be any of those which are specifically defined in said document.

The catalyst is employed in amounts ranging from as little as 0.001 moles/liter in the liquid phase, based on the alkylene carbonate reactant, to 0.1 mole/liter. Generally a concentration of from 0.01 to 0.05 mole/liter is preferred.

The primary materials contained in the homogeneous liquid phase in the conversion of alkylene carbonate to alkylene oxide is the alkylene carbonate reactant and a catalyst. A solvent is generally not necessary but if desired, an inert solvent such as tetraglyme or tetralin may be added to the liquid phase. This is particularly desirable in a continuous reaction system as a means of maintaining the catalyst in solution during catalyst recycle.

The process temperature of the reaction is generally maintained between 100°C and 250°C. The minimum temperature of the reaction is that temperature at which the catalytic decomposition of alkylene carbonate to alkylene oxide and carbon dioxide will occur. At temperatures below 100°C the rate of reaction of such carbonate decomposition is generally too low for practical consideration. Temperatures above 250°C are generally avoided so as to minimize the rate of reaction to undesired aldehyde and/or ketone by-products. For most effective operation of the process, a temperature range of from 170°C to 220°C is preferred.

Description of test procedure

The test reactor used in the following examples was a 75 ml Carious tube sealed at the top with a metal cap having ports for adding catalyst and alkylene carbonate to the reactor and sampling the effluent gas produced by the reaction. The test reactor was immersed in an oil bath to maintain a constant reaction temperature. The reactor was used in a continuous mode in which liquid alkylene carbonate was fed at a rate sufficient to maintain a constant liquid level. The effluent vapors leaving the reactor were passed into a 316 stainless steel manifold prior to being sampled and analyzed for ethylene oxide and acetaldehyde in a Perkin-Elmer model 990 gas chromatograph with a thermal conductivity detector. The manifold was connected to a vacuum pump so as to provide a sub-atmospheric pressure in the reactor. The effluent or off-gas of the reactor was condensed and collected using an acetone/dry ice condenser.

Doubly-distilled ethylene carbonate was distilled from calcium hydride prior to its use in the examples below. A fraction having a boiling point of from 128 to 129°C/165 mbar (124 mmHg) was collected. The ethylene carbonate thus recovered was weighed and introduced into the reactor which was maintained at the indicated reaction temperature by the oil bath.

Referring to the Table below, Example 1 was a control example in which a large amount of acetaldehyde (8400 ppm) by-product was formed at ambient pressure. In Example 2, the reaction was carried out under the nearly identical reaction conditions of Example 1 except that the reaction pressure was reduced to 133 mbar resulting in a greater than 80% reduction in acetaldehyde formation.

The effect of minimized aldehyde formation was also observed in Examples 3 and 4 which used a different reaction temperature and a different catalyst, respectively, relative to that used in Examples 1 and 2.

3

TABLE

Conversion of ethylene carbonate (EC) to ethylene oxide and $CO_2$ catalyzed by alkali metal salts

| Example | Catalyst | Catalyst concentration moles/l | Reaction temp. °C | Reaction pressure | Reaction time h | Rate of EC conversion mole/l · h | Acetaldehyde[1] ppm |
|---------|----------|-------------------------------|-------------------|-------------------|-----------------|----------------------------------|---------------------|
| 1 | NaBr | 0.066 | 180 | 1 bar | 6.0 | 1.4 | 8400 |
| 2 | NaBr | 0.072 | 180 | 133 mbar | 8.0 | 2.6 | 1400 |
| 3 | NaBr | 0.122 | 160 | 133 mbar | 8.0 | 0.7 | 1400 |
| 4 | LiBr | 0.018 | 180 | 133 mbar | 1.5 | 3.1 | 2200 |

[1] The ppm of acetaldehyde are relative to ethylene oxide.

# 0 047 473

**Claims**

1. A homogeneous catalytic process for converting an alkylene carbonate to the corresponding epoxide wherein a liquid phase comprising the alkylene carbonate and a soluble catalyst is heated to a temperature of from 100°C to 250°C at subatmospheric pressure, characterized in that heating of the liquid phase is performed at a pressure of 39.9 to 266 mbar (30 to 200 mmHg) and that the catalyst is an alkalimetal halide which is present in an amount of 0.0001 to 0.1 mole/l.

2. The process of claim 1, wherein the alkylene carbonate is ethylene carbonate.

**Patentansprüche**

1. Homogen katalytisches Verfahren zur Umsetzung eines Alkylencarbonats zu dem entsprechenden Epoxid, bei dem eine flüssige Phase enthaltend das Alkylencarbonat und einen gelösten Katalysator auf eine Temperatur von 100 bis 250°C bei Unterdruck erhitzt wird, dadurch gekennzeichnet, daß das Erhitzen der flüssigen Phase bei einem Druck von 39,9 bis 266 mbar (30 bis 200 mmHg) durchgeführt wird und der Katalysator ein Alkalihalogenid ist, das in einer Menge von 0,0001 bis 0,1 mol/l vorliegt.

2. Verfahren nach Anspruch 1, wobei das Alkylencarbonat Ethylencarbonat ist.

**Revendications**

1. Procédé de catalyse homogène pour convertir un carbonate d'alkylène en l'époxide correspondant par chauffage d'une phase liquide comprenant le carbonate d'alkylène et un catalyseur soluble à une temperature comprise entre 100°C et 250°C sous pression réduite, caracterisé en ce que le chauffage est exécuté sous une pression comprise entre 39,9 mbar et 266 mbar (30 et 200 mm Hg) et en ce que le catalyseur est un halogénure alkalin présent en une quantité comprise entre 0,0001 mole/l et 0,1 mole/l.

2. Procédé selon la revendication 1, caracterisé en ce que le carbonate d'alkylène est le carbonate d'éthylène.